(19) **Europäisches Patentamt**
**European Patent Office**
**Office européen des brevets**

(11) **EP 0 946 471 B2**

(12) **NOUVEAU FASCICULE DE BREVET EUROPEEN**
Après la procédure d'opposition

(45) Date de publication et mention de la décision concernant l'opposition:
**25.02.2009 Bulletin 2009/09**

(45) Mention de la délivrance du brevet:
**20.03.2002 Bulletin 2002/12**

(21) Numéro de dépôt: **97952068.1**

(22) Date de dépôt: **16.12.1997**

(51) Int Cl.:
***C07C 15/08*** (2006.01)  ***C07C 7/13*** (2006.01)

(86) Numéro de dépôt international:
**PCT/FR1997/002309**

(87) Numéro de publication internationale:
**WO 1998/028246 (02.07.1998 Gazette 1998/26)**

(54) **PROCEDE DE SEPARATION DE PARAXYLENE COMPRENANT UNE ADSORPTION AVEC INJECTION D'EAU ET UNE CRISTALLISATION**

VERFAHREN ZUR ABTRENNUNG VON PARA-XYLOL DAS EINE ABSORPTION MIT WASSERINJEKTION UND EINE KRISTALLISIERUNG ENTHÄLT

METHOD FOR SEPARATING PARAXYLENE COMPRISING ADSORPTION WITH WATER INJECTION AND CRYSTALLISATION

(84) Etats contractants désignés:
**DE ES FR GB IT NL SE**

(30) Priorité: **20.12.1996 FR 9615930**

(43) Date de publication de la demande:
**06.10.1999 Bulletin 1999/40**

(73) Titulaire: **Institut Français du Pétrole**
**92500 Rueil Malmaison (FR)**

(72) Inventeurs:
• **HOTIER, Gérard**
**F-69630 Chaponost (FR)**
• **METHIVIER, Alain**
**F-92500 Rueil Malmaison (FR)**
• **PUCCI, Annick**
**F-78290 Croissy sur Seine (FR)**

(74) Mandataire: **Renard, Emmanuelle**
**Cabinet Plasseraud**
**52 rue de la Victoire**
**75440 Paris Cedex 09 (FR)**

(56) Documents cités:
WO-A-96/20908       FR-A- 2 089 639
US-A- 3 734 974       US-A- 3 734 974
US-A- 3 755 153       US-A- 4 306 107
US-A- 4 442 222       US-A- 5 107 062
US-A- 5 284 992       US-A- 5 401 476

• **D. M. RUTHVEN: 'Principles of Adsorption and Adsorption Processes', 1984, JOHN WILEY & SONS, NEW YORK, US, ISBN 0-471-86606-7 pages 396 - 408**
• **A. J. DE ROSSET ET AL: 'Industrial Applications of Preparative Chromatography' 2000, pages 249 - 281**

**EP 0 946 471 B2**

Printed by Jouve, 75001 PARIS (FR)

**Description**

L'invention concerne un procédé de séparation de paraxylène à productibilité améliorée et à coût opératoire diminué, à partir d'un mélange d'isomères en C8 aromatiques en contenant.

Elle s'applique à la préparation de paraxylène de très haute pureté en vue de la synthèse d'acide téréphtalique, un intermédiaire dans l'industrie des nylons.

L'arrière plan technologique est décrit dans la demande FR-A-2 089 639.

On a décrit dans le brevet US 5401476 la combinaison de la séparation par adsorption en contre-courant simulé et de la cristallisation pour réaliser de manière plus économique qu'en une seule étape la production de paraxylène haute pureté. Les principes de base de cette combinaison sont les suivants :

- Dans l'étape de séparation en lit mobile simulé, on emploie des réglages différents de ceux de la production directe de paraxylène haute pureté. Le débit de charge est tel que les débits en zone 2 et zone 3 (avec débit de charge = débit zone 2 - débit zone 3) ne permettent plus la production simultanée d'un extrait contenant du paraxylène pur et d'un raffinat exempt de paraxylène. On augmente le débit de charge (donc la productivité ) en baissant le débit en zone 2. Il devient donc impossible d'obtenir du paraxylène pur (+ de 98 %). De plus, le débit de solvant est également diminué par rapport à un réglage « haute pureté » notamment en augmentant le débit en zone 4 au-delà du débit d'arrêt. L'augmentation de productivité et la baisse des coûts opératoires liés à l'emploi de moins de solvant se font au détriment de la pureté.

- Dans l'étape de cristallisation, on traite un mélange contenant de 75 % à 98 % de paraxylène et plus avantageusement de 85 à 95 % de paraxylène de manière à réutiliser les cristalliseurs préexistants des unités basées sur des schémas de cristallisation en deux étapes principales. On opère de préférence avec une température de cristallisation de - 15 °C à + 15 °C de manière à ne plus consommer de frigories à bas niveau thermique. Dans les cas les plus favorables, une synergie supplémentaire est trouvée en n'employant qu'un différents de ceux de la production directe de paraxylène haute pureté seul et même solvant comme désorbant dans l'étape d'adsorption et comme solvant de rinçage des cristaux dans l'étape de cristallisation. Cependant, lorsqu'on réalise l'étape d'adsorption de la charge sur l'adsorbant avec un rapport désorbant sur charge élevé, par exemple supérieur à 1,3, on atteint des performances en pureté du produit recherché très élevées, en général supérieures à 95 %. Plus la pureté du produit est élevée, plus elle peut fluctuer. On imagine donc aisément que dans la mesure où l'étape de purification par cristallisation, qui permet d'accéder à une pureté supérieure à 99 %, est calculée pour une charge de pureté déterminée et non soumise à des fluctuations, la marche du cristalliseur soit perturbée.

Le brevet US 3.734.974 décrit l'utilisation pour la séparation de paraxylène en lit mobile simulé (présumé de haute pureté en l'absence d'aucune mention sur du paraxylène basse pureté) de zéolithes X ou Y échangées avec des cations du groupe IA et/ou du groupe IIA (ou encore une association des 2) avec une teneur en eau contrôlée sur la zéolithe (de 1 à 5 %). Les désorbants utilisés sont par exemple le toluène, ou le paradiéthylbenzène ou les diethylbenzènes en mélange ou un mélange de ces constituants avec une coupe paraffinique. L'avantage de l'ajout d'eau, en particulier sur les zéolithes K BaX et BaX est une très nette augmentation des sélectivités paraxylène-ethylbenzène et paraxylène-metaxylène.

Le brevet US 4.778.946 décrit, pour la séparation éthylbenzène-metaxylène dans des charges exemptes de paraxylène, l'emploi de la zéolithe KY contenant jusqu'à 10 % d'eau et jusqu'à 4 % soit de méthanol soit d'ammoniac de manière non seulement à maximiser la sélectivité éthylbenzène-metaxylène mais encore à obtenir une sélectivité désorbant-éthylbenzène la plus proche possible de 1. Le ratio désorbant/charge utilisé est de 2/1. Ce document précise qu'un désorbant trop fortement adsorbé ne permet pas une bonne séparation et qu'un désorbant trop faiblement adsorbé entraîne une demande excessive en désorbant. Il recommande que la sélectivité éthylbenzène-métaxylène soit au moins égale à 3,0 et que la sélectivité éthylbenzène-désorbant soit comprise entre 1 et 2.

Le brevet US 5.401.476 ne suggère pas de travailler en présence d'eau, les hydrocarbures étant anhydres.

Le brevet US 3.734.974 ne suggère pas l'intérêt qu'il pourrait y avoir à injecter de l'eau dans un système où l'on ne cherche pas à obtenir du paraxylène de haute pureté et ne reconnaît pas le rôle que pourrait avoir l'eau sur la sélectivité paraxylène-désorbant.

Le brevet US 4.778.946 enseigne que l'eau peut modifier la sélectivité éthylbenzène-désorbant dans le cas de charges exemptes de paraxylène uniquement. Ni le brevet US 3.734.974, ni le brevet US 4.778.946 ne suggèrent de moyen simple et pratique non pas de contrôler la teneur en eau dans la zéolithe mais plutôt de rechercher à contrôler la teneur en eau dans les hydrocarbures en contact avec la zéolithe.

Les objets de l'invention sont de remédier aux inconvénients de l'art antérieur. De manière plus précise, le premier objet de l'invention est de réaliser un procédé de séparation de paraxylène haute pureté par combinaison d'une étape d'adsorption en lit mobile simulé et d'une étape de cristallisation, où l'étape d'adsorption en lit mobile simulé comporte la particularité de travailler à taux de solvant sur charge réduit grâce à une injection d'eau continue réalisée dans les courants qui alimentent la ou les colonnes d'adsorption.

Le second objet de l'invention est de changer de manière favorable la sélectivité paraxylène-désorbant de manière à obtenir une valeur optimale permettant de réduire la demande en désorbant. Ce qui est surprenant pour l'homme de l'art, c'est que l'injection d'eau ne produit pas une augmentation de pureté à rendement et productivité constants ni une augmentation de rendement à pureté et productivités fixées, ni une augmentation de productivité à rendement et pureté constants, mais une baisse de la demande en désorbant à pureté et productivité constantes.

Un troisième objet de l'invention est d'optimiser la teneur en eau dans les effluents hydrocarbonés en fonction de la nature de la zéolithe adsorbante et des cations de compensation et en fonction de la température.

On a observé qu'en introduisant une quantité appropriée d'eau dans les colonnes d'adsorption par l'intermédiaire des flux qui y entrent, en combinaison avec une quantité de désorbant limitée par rapport à la quantité de charge, on obtenait de très bons résultats.

Plus précisément, l'invention concerne un procédé de séparation de paraxylène à partir d'une charge comprenant un mélange d'isomères en C8 aromatiques en contenant, comportant une étape d'adsorption et de désorption des isomères du mélange, l'étape d'adsorption étant en lit mobile simulé, sur au moins une colonne contenant une zéolithe, l'étape d'adsorption et de désorption délivrant dans des conditions adéquates au moyen d'un désorbant choisi parmi le toluène et le paradiéthylbenzène (PDEB) une fraction riche en paraxylène et une fraction pauvre en paraxylène, le procédé comprenant en outre au moins une étape de cristallisation de la fraction riche en paraxylène qui délivre du paraxylène pur, le procédé étant **caractérisé en ce qu'**on introduit un débit d'eau dans la charge, dans le désorbant et/ou dans un courant de recyclage d'un flux dans la colonne par injection d'eau continue réalisée dans les courants qui alimentent la ou les colonnes d'adsorption et en ce que l'étape d'adsorption et de désorption est réalisé à une température de 140 à 160°C sur une zéolithe Y échangée par du baryum et du potassium, ledit débit d'eau étant tel que la moyenne pondérée des teneurs en eau, mesurée dans la fraction riche en paraxylène et dans la fraction pauvre en paraxylène, est comprise entre 3 et 6 ppm et le rapport S/F du débit de désorbant sur celui de la charge lors de l'étape d'adsorption et de désorption étant compris entre 1,15 et 1,35 ; ou

l'étape d'adsorption et de désorption est réalisée à une température de 165 à 185°C sur une zéolithe Y échangée par du baryum et du potassium, ledit débit d'eau étant tel que la moyenne pondérée des teneurs en eau, mesurée dans la fraction riche en paraxylène et dans la fraction pauvre en paraxylène, est comprise entre 6 et 12 ppm et le rapport S/F du débit de désorbant sur celui de la charge lors de l'étape d'adsorption et de désorption étant compris entre 1,10 et 1,35 ; ou

l'étape d'adsorption et de désorption est réalisée à une température de 165 à 185°C sur une zéolithe X échangée au baryum, ledit débit d'eau étant tel que la moyenne pondérée des teneurs en eau, mesurée dans la fraction riche en paraxylène et dans la fraction pauvre en paraxylène, est comprise entre 90 et 110 ppm, et le rapport S/F du débit de désorbant sur celui de la charge lors de l'étape d'adsorption et de désorption étant compris entre 0,95 et 1,2.

On a constaté des résultats optimum, résultant de la combinaison ci-dessus en fonction de la nature de la zéolithe, de ses cations de compensation et de la température opératoire.

C'est ainsi que selon un premier mode de mise en oeuvre du procédé, on peut réaliser l'étape d'adsorption et de désorption à une température de 140 à 160°C sur une zéolithe Y échangée par du baryum et du potassium, la dite moyenne pondérée des teneurs en eau étant comprise entre 3 et 6 ppm et le rapport S/F étant compris entre 1,15 et 1,35.

Selon un deuxième mode de mise en oeuvre, on peut réaliser l'étape d'adsorption et de désorption à une température de 165 à 185 °C sur une zéolithe Y échangée par du baryum et du potassium, ladite moyenne pondérée des teneurs en eau étant comprise entre 6 et 12 ppm et le rapport S/F étant compris entre 1,10 et 1,35.

Selon un troisième mode de mise en oeuvre, on peut réaliser l'étape d'adsorption et de désorption à une température de 165 à 185 °C sur une zéolithe X échangée au baryum, ladite moyenne pondérée des teneurs en eau étant comprise entre 90 et 110 ppm et le rapport S/F étant compris entre 0,95 et 1,2.

Il est bien admis que la fraction riche en paraxylène et la fraction pauvre en paraxylène peuvent être distillés pour les débarrasser du désorbant. La fraction pauvre en solvant débarrassée du désorbant contenant éventuellement de l'eau en quantité minime peut être isomérisée selon des procédés d'isomérisation bien connus et l'isomérat enrichi en paraxylène, éventuellement débarrassé des composés légers peut être recyclé, au moins en partie, vers la zone d'adsorption et de désorption.

Selon une caractéristique du procédé, la fraction riche en paraxylène peut être cristallisée, le plus souvent à haute température, par exemple supérieure à -30°C selon les brevets US 5.401.476 et WO.96-20.908, décrivant des étapes de cristallisation à un ou plusieurs niveaux de température.

Le solvant généralement utilisé est le toluène ou le paradiéthylbenzène par exemple. Il peut être recyclé au moins en partie vers l'étape d'adsorption et de désorption, sensiblement anhydre.

Selon une autre caractéristique, il peut être avantageux d'introduire du méthanol dans une proportion généralement inférieure à 500 ppm, dans la fraction riche en paraxylène débarrassée du désorbant mais contenant une quantité minime d'eau, qui est destinée à être cristallisée. L'étape de cristallisation délivre alors une liqueur mère contenant de l'eau et du méthanol que l'on retire par exemple par adsorption, et l'on recycle au moins en partie ladite liqueur mère ainsi débarrassée de l'eau et du méthanol à l'étape d'adsorption et de désorption.

Les teneurs en eau dans les phases hydrocarbonées sont naturellement reliées aux teneurs en eau sur le zéolithe par un isotherme d'adsorption qui ne dépend sensiblement pas de la nature de l'hydrocarbure adsorbé. Comme le signale l'art antérieur, il faut distinguer entre l'eau adsorbée sur la zéolithe que l'on mesure par une perte au feu à 400 °C et que l'art antérieur mesure par une perte au feu à 500 °C (dénommée « Relative Volatile Free Basis ») et entre l'eau beaucoup plus fortement retenue qui est mesurée par une perte au feu à 900 °C ou 1 000 °C « Loss on ignition ». Lors de la mesure à 900 °C ou 1 000 °C, la structure de la zéolithe est détruite et l'on peut considérer que la différence d'eau désorbée entre 400 °C et 1 000 °C représente de l'eau de constitution. La différence entre ces deux termes est de l'ordre de 1,5 à 2 % poids sur les faujasites.

On réalise la mesure de l'isotherme d'adsorption d'eau de la façon suivante : on laisse s'hydrater à l'atmosphère ambiante un lot de solide à tester. On remplit plusieurs colonnes d'un mètre de longueur et d'un centimètre de diamètre (78,5 cm3) de cette zéolithe et l'on dispose ces colonnes dans un four à 250 °C sous flux d'azote très sec (moins de 10 ppm d'eau). On laisse se déshydrater chacune de ces colonnes pendant des temps différents de manière à obtenir diverses teneur en eau (que l'on mesure par pesée).

Chaque colonne est ensuite disposée dans une boucle fermée comprenant une réserve d'hydrocarbure sec (de volume minimum), une pompe volumétrique à piston, pour chromatographie liquide et la colonne. La réserve d'hydrocarbure est équipée d'une prise d'échantillon de manière à mesurer la teneur en eau de l'hydrocarbure une fois l'équilibre atteint. La quantité totale d'hydrocarbure est de 100 cm3. De cette manière, la quantité d'eau contenue dans l'hydrocarbure est négligeable en regard de celle restant retenue sur la zéolithe.

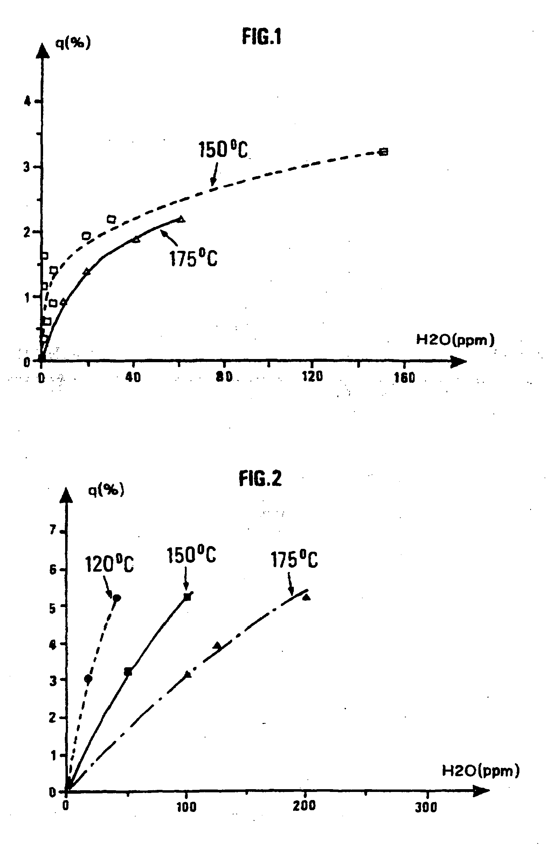

On obtient, ainsi, les isothermes d'adsorption présentées en figures 1 et 2 relatives respectivement aux zéolithes KBaY et BaX mesurées par la perte au feu à 400 °C représentant la quantité d'eau (%) adsorbée par les zéolithes en fonction de la concentration (ppm) en eau dans la phase hydrocarbonée à différentes températures.

L'influence de la teneur en eau sur la séparation par adsorption est étudiée complètement indépendamment de la mesure de l'isotherme. De cette manière, on évite d'avoir à tenir des bilans cumulatifs des entrées et des sorties d'eau dans l'unité de séparation sur lesquels les erreurs cumulées sont souvent très importantes.

L'unité de séparation est remplie de zéolithe préalablement activée en four tournant au moment de la phase finale de fabrication. Suivant les lots de fabrication, la perte au feu à 400°C de la zéolithe avant chargement est comprise entre 2 % et 5 %. Au cours du chargement, l'adsorbant se réhydrate en partie à un degré indéterminé (toujours inférieur à 7 %).

Lorsque l'on veut travailler en conditions anhydres, on fait passer sur la zéolithe du désorbant sec jusqu'à ce que l'on ne mesure plus que des teneurs en eau inférieures à 1 ppm sur les effluents de l'unité. Cette opération requiert un temps important (2 à 3 semaines).

Au contraire, lorsque l'on veut travailler à teneur en eau connue, on procède en injectant deux débits contrôlés de désorbant, l'un de produit anhydre, l'autre de produit saturé en eau et un débit contrôlé de charge anhydre (on peut également effectuer l'inverse). Par exemple, si l'on souhaite contrôler une teneur moyenne en eau de 50 ppm sur les flux entrants dans l'unité avec des débits respectifs de 5 cm3/min. de charge et 7 cm3/min. de désorbant, on injecte 5 cm3/min. de charge anhydre, 5,6 cm3/min. de solvant anhydre et 1,4 cm3/min. de solvant saturé en eau à température ambiante (430 ppm dans le cas du toluène).

On maintient ces conditions jusqu'à ce que la moyenne pondérée des teneurs en eau sur les sorties soit substantiellement 50 ppm ; par exemple, si l'on obtient des teneurs en eau respectives de 43 ppm sur un débit d'extrait de 5,3 cm3 min. et de 54 ppm sur un débit de raffinat de 6,65 cm3, la moyenne pondérée sur les sorties est de 49 ppm ce qui est considéré comme acceptable compte tenu de la précision des mesures de teneur en eau.

Pour mesurer les teneurs en eau sur les flux entrants et sortants, on utilise la méthode de KARL FISCHER pour des teneurs supérieures à 15 ppm. Lorsque ces teneurs sont inférieures à 15 ppm, on se fie aux mesures en ligne délivrées par les sondes d'analyse en ligne (appareil PANAMETRIC, séries 1). Un étalonnage est réalisé entre 15 ppm et 200 ppm, l'extrapolation de cette courbe d'étalonnage pour des teneurs comprises entre 1 et 15 ppm est considérée comme valide.

Les exemples suivants décrivent de manière comparative l'utilisation du système zéolithe KBaY-toluène avec et sans eau, du système zéolithe KBaY-PDEB avec ou sans eau, du système BaX-toluène avec et sans eau et du système BaX-PDEB avec et sans eau. Les figures annexées sont les suivantes :

- les figures 1 et 2 représentent la quantité d'eau adsorbée par diverses zéolithes en fonction de la concentration (ppm) en eau dans la phase hydrocarbonée, à différentes températures ;
- les figures 3, 4 et 5 montrent le taux de solvant à performances constantes en fonction de la moyenne pondérée des quantités d'eau (ppm) contenues dans les sorties (extrait et raffinat), à différentes températures ;
- les figures 6, 7 et 8 montrent un indice de performance en fonction de la moyenne pondérée des teneurs en eau dans les sorties, exprimée en ppm ; et
- la figure 9 représente in indice de performance relatif à divers désorbants en fonction du rapport désorbant sur charge (S/F).

Exemple n°1

On a réalisé une unité pilote de chromatographie liquide continue à partir de 24 colonnes en série de 1 m de longueur et de 1 cm de diamètre, la circulation entre la 24ème et la première colonne se faisant au moyen d'une pompe de recyclage. À chaque liaison intercolonne, on peut injecter soit une charge à séparer soit du solvant. On peut également soutirer soit un raffinat soit un extrait. Cette unité est décrite dans un ouvrage intitulé « Preparative and production scale chromatography processes with applications », édité par G. Barker, G. Ganestsos, chapitre « From batch elution to simulated counter current chromatography » par B. Balannec et G. Hotier, (Publication de Marcel Dekker Inc., New-York 1992). L'adsorbant est constitué de zéolithe Y échangée par du potassium et par du baryum, le taux d'échange exprimé en normalité est d'environ 50 % pour chacun des deux cations. La zéolithe est mise en oeuvre sous forme de billes de 0,315 à 0,5 mm de diamètre. L'ensemble des colonnes et du vannage de distribution est placé dans une étuve à 150 °C.

Suivant le principe de la chromatographie à contre-courant simulé, on avance de trois colonnes toutes les six minutes à co-courant de la circulation de liquide, l'injection de solvant, le prélèvement d'extrait, l'injection de la charge et le prélèvement de raffinat.

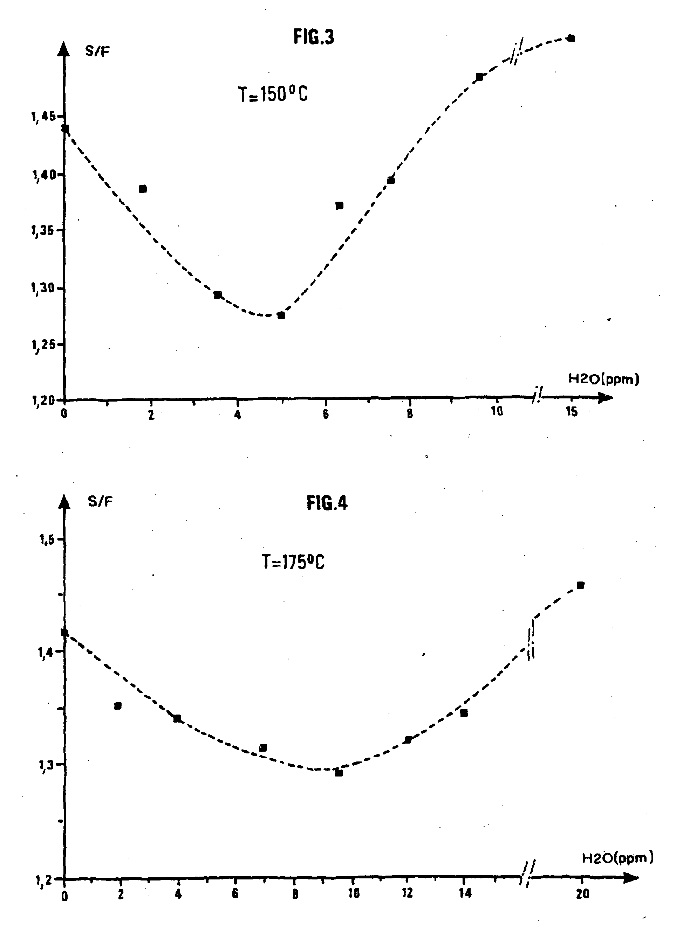

Selon l'invention, le nombre de lits à considérer est donc de huit. Six colonnes (donc deux lits) sont comprises entre l'injection de solvant et le prélèvement d'extrait, neuf colonnes (trois lits) sont comprises entre le prélèvement d'extrait et l'injection de charge, trois colonnes (un lit) sont comprises entre l'injection de charge et le prélèvement de raffinat et les six dernières colonnes (deux lits) se situent entre le prélèvement de raffinat et l'injection de solvant. Les performances de l'unité en lit mobile simulé sont observées en fonction de la moyenne pondérée des teneurs en eau de l'extrait et du raffinat, l'eau ayant été introduite de manière continue dans les colonnes par l'intermédiaire de désorbant anhydre et de désorbant saturé comme décrit ci-dessus. Pour le premier point de la courbe de la figure 3, on rappelle les conditions de l'exemple n°1 de US 5.401.476. On injecte en continu (exprimé aux conditions ambiantes) 7,2 cm3/min de toluène et 5 cm3/min de charge. On prélève également en continu 5,40 cm3/min d'extrait et 6,74 cm3/min de raffinat ; on constate environ 5 % de pertes. Pendant la première période du cycle qui en compte huit, le solvant est injecté en colonne 1, l'extrait est prélevé à la sortie de la colonne 6, la charge est injecté en colonne 15, le raffinat est prélevé à la sortie de la colonne 18. Pendant les deux première périodes du cycle, la pompe de recyclage débite à température ambiante 38,7 cm3/min ; pendant la troisième période, elle débite à 45,5 cm3/min ; pendant les trois périodes suivantes elle débite à 40,5 cm3/min et pendant les deux dernières périodes elle débite 45,9 cm3/min. On a donc un débit de recyclage moyen de 42 cm3/min. Le paraxylène est obtenu avec une pureté de 92,2 % et un taux de récupération de 98,1 %. La pression décroît à peu près linéairement de 30 bar à 5 bar. Le tableau suivant donne le bilan en régime stationnaire de l'unité :

|  | Charge | Solvant | Extrait | Raffinat |
|---|---|---|---|---|
| Débit | 5 cm3/min | 7,2 cm3/min | 5,40 cm3/min | 6,74 cm3/min |
| Toluène |  | 99,9 % | 79,30 % | 43,29 % |
| Ethylbenzène | 17 % |  | 1,07 % | 11,72 % |
| M Xylène | 4 % |  | 0,40 % | 32,30% |
| O Xylène | 18% |  | 0,15% | 12,40% |
| P Xylène | 21 % |  | 19,08 % | 0,29% |

Une première manière d'opérer l'unité consiste à réduire le débit de désorbant (toluène) tout en gardant constant le débit de charge. On s'efforce de maintenir à peu près constants la pureté et le rendement ; ceci est réalisé en jouant sur la balance extrait-raffinat, les débits dans les zones 2 et 3 ainsi que le temps des permutations sont maintenus constants, les débits dans les zones 1 et 4 sont ajustés par augmentation du débit en zone 4 et diminution du débit en zone 1.

Cette manière d'opérer se rapproche du type d'opération sur une unité industrielle, cependant il est difficile d'obtenir des points à isopureté et isorendement. On tolère des variations de pureté et de rendement de l'ordre de 1 % à condition que la moyenne géométrique de la pureté et du rendement ne varie pas plus que 0,3 %.

La figure 3 montre que le débit de solvant est minimisé à 150 °C pour une teneur en eau moyenne de 5 ppm : le ratio solvant sur charge est de 1,27/1 alors que pour un solvant anhydre (cas de US 5.401.476), il s'établit à 1,44/1.

Exemple 2 (fig. 4)

L'expérience est reprise en augmentant la température opératoire de 150 °C à 175 °C. Les performances de pureté et de rendement sont maintenues identiques par rapport aux expériences à 150 °C pour des taux de solvant presque identiques pour la zéolithe anhydre et pour l'optimum de teneur en eau. Cependant, la figure 4 montre que la teneur en eau optimale n'est pas de 5 ppm mais 10 ppm.

Ceci correspond à un optimum de teneur en eau sur la zéolithe de 0,4 à 0,5 % (perte au feu à 400 °C), ce qui est impossible à mesurer avec une précision correcte directement.

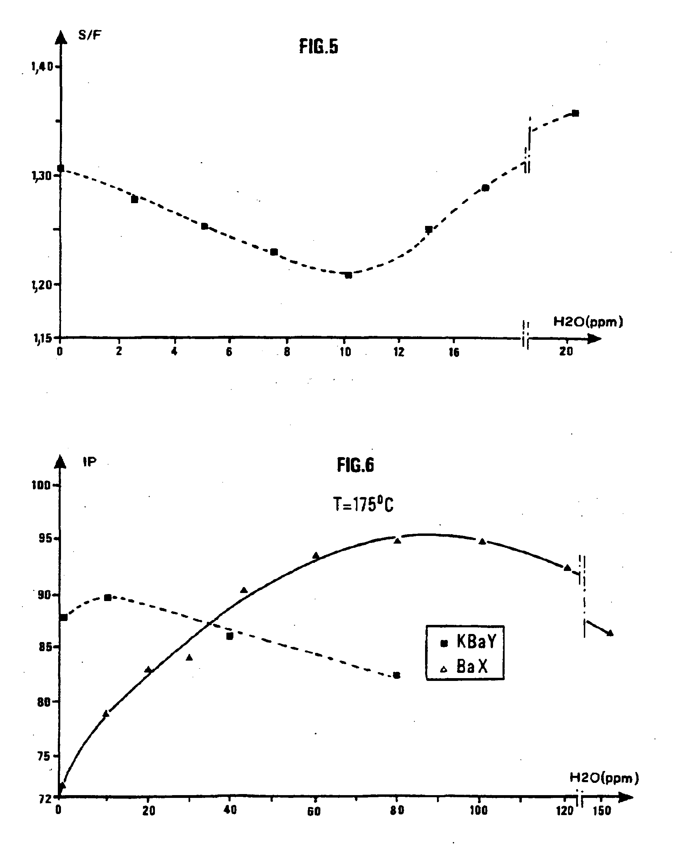

Exemple 3 (fig. 5)

L'expérience à 175 °C est répétée avec du désorbant paradiéthylbenzène à 98 % de pureté (les impuretés sont constituées majoritairement de meta et d'orthodiéthylbenzène). La figure 5 montre que le taux de solvant peut être réduit d'environ 0,1/1 et que l'optimum de performances se situe encore à 10 ppm d'eau dans l'hydrocarbure. Ce gain en taux de solvant est à relier à la sélectivité PX-désorbant qui est de 0,55 dans le cas du toluène et 0,72 dans le cas du paradiéthylbenzène.

Exemple 4 (fig. 6)

La même unité est chargée avec de la zéolithe BaX sous forme de billes de 0,3 à 0,8 mm de diamètre contenant 22 % de liant à base d'argile. La teneur résiduelle en sodium après échange est inférieure à 3 % des cations (exprimé en normalité).

On emploie seulement 12 colonnes au lieu de 24 et les permutations se font toutes les colonnes au lieu de toutes les 3 colonnes comme précédemment. On compte 3 lits dans chacune des zones. Le temps de permutation est une minute, le ratio entre le débit de recyclage moyen et la charge est de 4,5/1, le taux de solvant est gardé constant à 1,25/1, le ratio extrait sur raffinat est de 0,40/1 et le débit de charge est de 8 cm3/min. La productivité est beaucoup plus importante par rapport au cas précédant grâce à une réduction de la perte de charge. L'unité est opérée à 175 °C et le désorbant est du paradiéthylbenzène.

On définit un indice de performances : $IP = \sqrt{\text{rendement \% x Pureté \%}}$. Les débits dans les zones sont légèrement ajustés de manière à garder une balance raisonnable entre pureté et rendement (jamais plus de 6 % d'écart). La figure 6 montre que l'indice de performance sur la zéolithe BaX est maximum pour une teneur en eau dix fois plus forte environ qu'avec la zéolithe KBaY ; d'autre part, les performances de la zéolithe BaX sont nettement inférieures à celles de la zéolithe KBaY lorsque ces produits sont anhydres alors qu'elles sont nettement supérieures, à l'optimum de teneur en eau, ce qui n'est nullement enseigné par l'art antérieur.

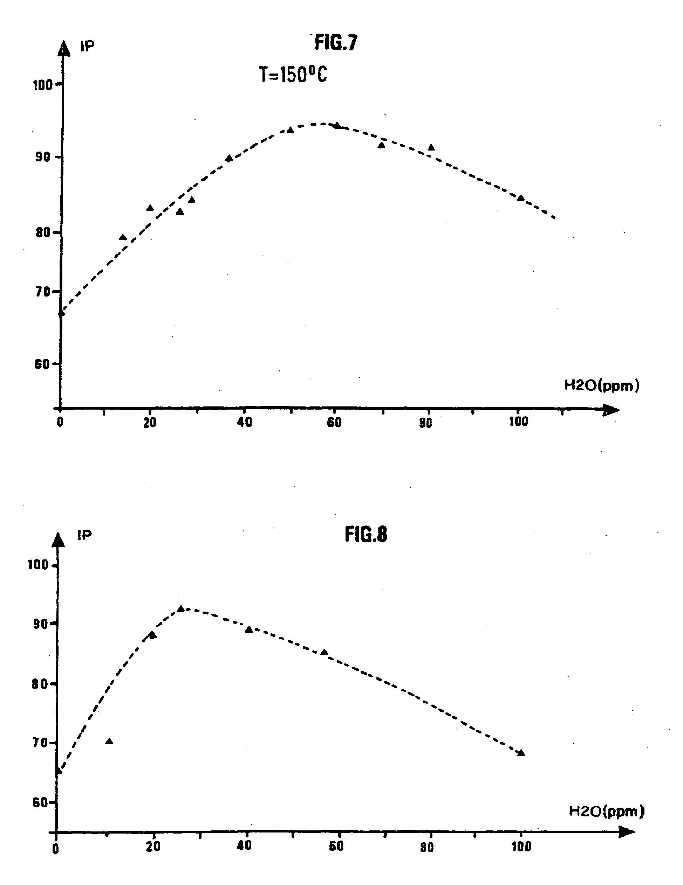

Exemple (fig. 7)

La même expérience est répétée à 150 °C. Outre une perte de charge considérablement plus forte (augmentation d'environ 70 %), on note des performances plus faibles et un optimum différent de teneur en eau dans l'hydrocarbure (50 à 60 ppm (voir fig. 7)).

Exemple 6 (fig. 8)

Les exemples 4 et 5 sont répétés sauf que le solvant est du toluène et la température de 120 °C : la performance maximale est obtenue cette fois pour une teneur en eau dans l'hydrocarbure (moyenne pondérée des sorties) d'environ 25 ppm, le ratio extrait sur raffinat est d'environ 0,7/1 (voir fig. 8).

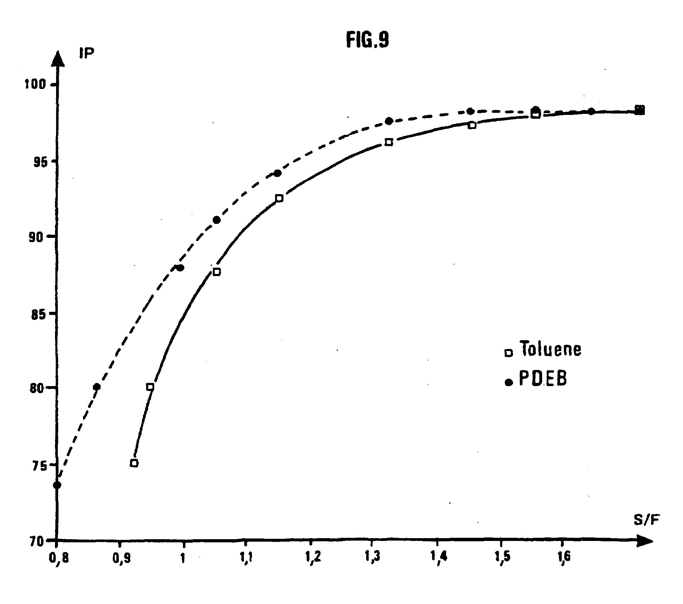

Exemple 7 (fig. 9)

On reprend l'exemple 4 en fixant cette fois-ci la teneur en eau à l'optimum soit 90 ppm et l'on fait varier le ratio solvant/charge. La charge ne contient que 4 % d'éthylbenzène pour 22,5 % de paraxylène. On compte en outre 23,5 % d'orthoxylène, 1,5 % de toluène et 48,5 % de métaxylène. Cette charge étant plus facile à traiter, on peut en injecter un débit supérieur soit 10 cm3/min. Le ratio recycle sur charge est baissé à 4,4/1 et le temps de permutation est baissé à 50 secondes. On observe qu'au-dessus d'un ratio solvant sur charge de 1,45/1, les performances demeurent identiques. En baissant le débit de désorbant jusqu'à 8 cm3/min., on réalise qu'il est possible, pour des taux de solvant inférieurs à 1, d'obtenir encore des puretés supérieures à 75 % suffisantes pour une purification par cristallisation qui constitue la seconde étape du procédé (voir fig. 9).

Lorsque l'on remplace le désorbant PDEB par du toluène, on réalise que les performances sont légèrement moins bonnes et que les réglages de débits sont différents (les débits en zone 1 et 4 sont plus forts et le ratio extrait sur raffinat doit être augmenté. L'intérêt d'utiliser le toluène est de pouvoir traiter des charges contenant de fortes teneurs en C9 aromatiques.

Suivant que l'on utilise le toluène ou le paradiéthylbenzène, l'eau contenue dans l'extrait devra être retirée avant d'envoyer le paraxylène basse pureté à l'étape de cristallisation.

Lorsque le désorbant utilisé est le toluène, le paraxylène impur est soutiré (sensiblement exempt d'eau) en fond de la colonne d'extrait. En tête des colonnes à distiller d'extrait et de raffinat, on soutire de l'eau liquide grâce à un plateau décanteur et l'on réalise en continu ou périodiquement des purges de constituants plus légers que le toluène. Environ 3 à 10 plateaux plus bas, on soutire le toluène sensiblement anhydre sur un plateau soutireur.

Lorsque le désorbant est le paradiéthylbenzène et que la charge à traiter contient un peu de toluène, il est également possible d'opérer de cette manière (dans ce cas en tête de colonne à distiller, on soutire de l'eau et du toluène) et le paraxylène impur est soutiré sensiblement anhydre, environ 10 plateaux en dessous.

On peut également ne pas utiliser cette zone de pasteurisation, refroidir le paraxylène, décanter l'eau vers 10 °C puis envoyer le paraxylène saturé en eau dans la cristallisation. Dans ce cas, si la cristallisation est opérée à une température inférieure à 0 °C, on injecte en général du méthanol pour éviter l'accumulation de glace dans les équipements de cristallisation. Avant de réinjecter la liqueur mère de cristallisation à l'étape d'adsorption, il faut enlever l'eau et le méthanol par adsorption ou par distillation.

Quant au raffinat débarrassé de solvant, il peut contenir sans inconvénient pour le catalyseur d'isomérisation jusqu'à 200 ppm d'eau.

## Revendications

**1.** Procédé de séparation de paraxylène à partir d'une charge comprenant un mélange d'isomères en C8 aromatiques en contenant, comportant une étape d'adsorption et de désorption des isomères du mélange, l'étape d'adsorption étant en lit mobile simulé, sur au moins une colonne contenant une zéolithe, l'étape d'adsorption et de désorption délivrant dans des conditions adéquates au moyen d'un désorbant choisi parmi le toluène et le paradiéthylbenzène (PDEB) une fraction riche en paraxylène et une fraction pauvre en paraxylène, le procédé comprenant en outre au moins une étape de cristallisation de la fraction riche en paraxylène qui délivre du paraxylène pur, le procédé étant **caractérisé en ce qu'**on introduit un débit d'eau dans la charge, dans le désorbant et/ou dans un courant de recyclage d'un flux dans la colonne par injection d'eau continue réalisée dans les courants qui alimentent la ou les colonnes d'adsorption,
et **en ce que**
l'étape d'adsorption et de désorption est réalisée à une température de 140 à 160°C sur une zéolithe Y échangée par du baryum et du potassium, ledit débit d'eau étant tel que la moyenne pondérée des teneurs en eau, mesurée dans la fraction riche en paraxylène et dans la fraction pauvre en paraxylène, est comprise entre 3 et 6 ppm et le rapport S/F du débit de désorbant sur celui de la charge lors de l'étape d'adsorption et de désorption étant compris entre 1,15 et 1,35 ; ou
l'étape d'adsorption et de désorption est réalisée à une température de 165 à 185°C sur une zéolithe Y échangée par du baryum et du potassium, ledit débit d'eau étant tel que la moyenne pondérée des teneurs en eau mesurée dans la fraction riche en paraxylène et dans la fraction pauvre en paraxylène, est comprise entre 6 et 12 ppm et le rapport S/F du débit de désorbant sur celui de la charge lors de l'étape d'adsorption et de désorption étant compris entre 1,10 et 1,35 ; ou
l'étape d'adsorption et de désorption est réalisée à une température de 165 à 185°C sur une zéolithe X échangée au baryum, ledit débit d'eau étant tel que la moyenne pondérée des teneurs en eau mesurée dans la fraction riche en paraxylène et dans la fraction pauvre en paraxylène, est comprise entre 90 et 110 ppm, et le rapport S/F du débit de désorbant sur celui de la charge lors de l'étape d'adsorption et de désorption étant compris entre 0,95 et 1,2.

**2.** Procédé selon la revendication 1, dans lequel la fraction riche en paraxylène et la fraction pauvre en paraxylène qui résultent de l'étape d'adsorption et de désorption sont distillées pour les débarrasser du désorbant, la fraction riche en paraxylène sensiblement débarrassée de désorbant étant soumise à l'étape de cristallisation.

**3.** Procédé selon la revendication 2, dans lequel le désorbant est recyclé en partie au moins sensiblement anhydre à l'étape d'adsorption et de désorption.

**4.** Procédé selon l'une des revendications 2 à 3, dans lequel on introduit du méthanol dans la fraction riche en paraxylène débarrassée du désorbant, mais contenant une quantité minime d'eau, avant l'étape de cristallisation.

**5.** Procédé selon la revendication 4, dans lequel l'étape de cristallisation délivre une liqueur mère contenant de l'eau et du méthanol et dans lequel on retire l'eau et le méthanol de la liqueur mère, avant de la recycler au moins en partie à l'étape d'adsorption et de désorption.

**6.** Procédé selon l'une des revendications 2 à 5, dans lequel la fraction pauvre en paraxylène débarrassée du désorbant est isomérisée.

**Claims**

**1.** A process for separating para-xylene from a feed comprising a mixture of aromatic $C_8$ isomers containing para-xylene, comprising a step for adsorption and desorption of isomers in the mixture, the adsorption step being in simulated moving bed, in at least one column containing a zeolite, the adsorption and desorption step delivering, under suitable conditions by means of, at least one desorbent, chosen from toluene or paradiethylbenzene (PDEB), a fraction which is rich in para-xylene and a fraction which is depleted in para-xylene, the process further comprising at least one step for crystallizing the fraction which is rich in para-xylene which delivers pure para-xylene, the process being **characterized in that** a stream of water is introduced into the feed, into the desorbent and/or into a recycling stream for one flux in the column, by continuous injection of water conducted into the streams which feed the adsorption column(s) and **in that**

the adsorption and desorption step is carried out at a temperature of 140°C to 160°C with a Y zeolite exchanged with barium and potassium, said water flow rate being such that the weighted average of the water contents measured in the fraction which is rich in para-xylene and in the fraction which is depleted in para-xylene is in the range 3 to 6 ppm and the S/F ratio of the flow rate of the desorbent to that of the feed during the adsorption and desorption step is in the range 1.15 to 1.35; or

the adsorption and desorption step is carried out at a temperature of 165°C to 185°C with a Y zeolite exchanged with barium and potassium, said water flow rate being such that the weighted average of the water contents measured in the fraction which is rich in para-xylene and in the fraction which is depleted in para-xylene is in the range 6 to 12 ppm and the S/F ratio of the flow rate of the desorbent to that of the feed during the adsorption and desorption step is in the range 1.10 to 1.35; or

the adsorption and desorption step is carried out at a temperature of 165°C to 185°C with an X zeolite exchanged with barium, said water flow rate being such that the weighted average of the water contents measured in the fraction which is rich in para-xylene and in the fraction which is depleted in para-xylene is in the range 60 to 130 ppm, preferably in the range 90 to 110 ppm, and the S/F ratio of the flow rate of the desorbent to that of the feed during the adsorption and desorption step is in the range 0.95 to 1.2.

**2.** A process according to claim 1, in which the fraction which is rich in para-xylene and the fraction which is depleted in para-xylene from the adsorption and desorption step are distilled to free them of desorbent, the fraction which is rich in para-xylene and substantially free of desorbent undergoing the crystallization step.

**3.** A process according to claim 2, in which the desorbent is recycled in part to the adsorption and desorption step in a substantially anhydrous state.

**4.** A process according to claim 2 or claim 3, in which methanol is introduced into the fraction which is rich in para-xylene free of desorbent but containing a minimum quantity of water, before the crystallization step.

**5.** A process according to claim 4, in which the crystallization step delivers a mother liquor containing water and methanol and in which the water and methanol are removed from the mother liquor before recycling it at least in part to the adsorption and desorption step.

**6.** A process according to any one of claims 2 to 5, in which the fraction which is depleted in para-xylene and free of desorbent is isomerised.

**Patentansprüche**

**1.** Verfahren zur Abtrennung von para-Xylol aus einem Einsatzprodukt [feed], welches eine Mischung aus aromatischen $C_8$-Isomeren umfasst, die para-Xylol enthalten, wobei das Verfahren einen Schritt zur Adsorption und Desorption von Isomeren in der Mischung umfasst, wobei der Adsorptionsschritt in einem simulierten Bewegtbett stattfindet, in mindestens einer Säule, die einen Zeolithen enthält, wobei der Adsorptions- und Desorptionsschritt unter geeigneten Bedingungen mittels mindestens eines Desorbens, welches ausgewählt ist aus Toluol oder para-Diethylbenzol (PDEB), eine Fraktion, die reich ist an para-Xylol und eine Fraktion, die verarmt ist an para-Xylol, liefert, wobei das Verfahren weiterhin mindestens einen Schritt zur Kristallisation der Fraktion umfasst, welche reich ist an para- Xylol, wobei das Verfahren **dadurch gekennzeichnet ist, dass** ein Wasserstrahl in das Einsatzprodukt, in das Desorbens und/oder in einen Recyclingstrom für einen Durchfluss der Säule [for one flux in the column] eingeführt wird, mittels kontinuierlicher Injektion von Wasser, welches in die Ströme eingeleitet wird, welche die Adsorptionssäule(n) speisen und

**dadurch** dass

der Adsorptions- und Desorptionsschritt bei einer Temperatur von 140°C bis 160°C mit einem Y-Zeolithen, der mit Barium und Kalium ausgetauscht ist, durchgeführt wird, wobei die Wasserflussrate so (gewählt) ist, dass der gewichtete Mittelwert der Wassergehalte, welche in der Fraktion, die reich an para-Xylol ist und in der Fraktion, die an para-Xylol verarmt ist, gemessen werden, im Bereich von 3 bis 6 ppm liegt und das S/F-Verhältnis der Flussrate des Desorbens zu der des Einsatzproduktes während des Adsorptions- und Desorptionsschrittes im Bereich von 1,15 bis 1,35 liegt; oder

der Adsorptions- und Desorptionsschritt bei einer Temperatur von 165°C bis 185°C mit einem Y-Zeolithen, der mit Barium oder Kalium ausgetauscht ist, durchgeführt wird, wobei die Wasserflussrate so (gewählt) ist, dass der gewichtete Mittelwert der Wassergehalte, welche in der Fraktion, die reich an pare-Xylol ist und in der Fraktion, die an para-Xylol verarmt ist, gemessen werden, im Bereich von 6 bis 12 ppm liegt und das S/F-Verhältnis der Flussrate des Desorbens zu der des Einsatzproduktes während des Adsorptions- und Desorptionsschrittes im Bereich von 1,10 bis 1,35 liegt; oder

der Adsorptions- und Desorptionsschritt bei einer Temperatur von 165°C bis 185°C mit einem X-Zeolith, der mit Barium ausgetauscht ist, durchgeführt werden, wobei die Wasserflussrate so (gewählt) ist, dass der gewichtete Mittelwert der Wassergehalte, die in der Fraktion, welche reich an para-Xylol ist und in der Fraktion, welche an para-Xylol verarmt ist, gemessen werden, im Bereich von 60 bis 130 ppm, vorzugsweise im Bereich von 90 bis 110 ppm, liegt und das S/F-Verhältnis der Flussrate des Desorbens zu der des Einsatzproduktes während des Adsorptions- und Desorptionsschrittes im Bereich von 0,95 bis 1,2 liegt.

2. Verfahren nach Anspruch 1, wobei die Fraktion, welche durch den Adsorptions- und Desorptionsschritt reich an para-Xylol ist und die Fraktion, welche an para-Xylol verarmt ist, destilliert werden, um sie vom Desorbens zu befreien, wobei die Fraktion, welche reich an para-Xylol und im Wesentlichen frei von Desorbens ist, dem Kristallisationsschritt unterworfen wird.

3. Verfahren nach Anspruch 2, wobei das Desorbens in einem im Wesentlichen wasserfreien Zustand teilweise dem Adsorptions- und Desorptionsschritt wiederzugeführt wird.

4. Verfahren nach Anspruch 2 oder 3, wobei der Fraktion, welche reich an para-Xylol und frei von Desorbens ist, aber ein Minimum an Wasser enthält, vor dem Kristallisationsschritt Methanol zugesetzt wird.

5. Verfahren nach Anspruch 4, wobei der Kristallisationsschritt eine Mutterlauge liefert, die Wasser und Methanol enthält und wobei das Wasser und Methanol vor der zumindest teilweisen Rückführung zum Adsorptions- und Desorptionsschritt aus der Mutterlauge entfernt werden.

6. Verfahren nach einem der Ansprüche 2 bis 5, wobei die Fraktion, welche an para-Xylol verarmt und frei von Desorbens ist, isomerisiert wird.

FIG.1

FIG.2

FIG.3

FIG.4

FIG.5

FIG.6

T=175°C

KBaY
BaX

## FIG.7

T=150°C

IP

H2O(ppm)

## FIG.8

IP

H2O(ppm)

FIG.9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2089639 A
- US 5401476 A
- US 3734974 A
- US 4778946 A
- WO 9620908 A

**Littérature non-brevet citée dans la description**

- Preparative and production scale chromatography processes with applications. **B. BALANNEC ; G. HOTIER.** From batch elution to simulated counter current chromatography. Marcel Dekker Inc, 1992